# EUROPEAN PATENT SPECIFICATION

(11) **EP 3 447 058 B1**
(45) Date of publication and mention of the grant of the patent: **11.10.2023**
(21) Application number: 17785382.7
(22) Date of filing: 13.04.2017
(51) Int. Cl.: C07F 5/02, A61K 31/69, A61P 31/04

(54) **NOVEL BROAD-SPECTRUM BETA-LACTAMASE INHIBITOR**
NEUARTIGER BREITSPEKTRUM BETA-LAKTAMASE-INHIBITOR
NOUVEL INHIBITEUR DE LA BETA-LACTAMASE À SPECTRE LARGE

(30) Priority: 19.04.2016 CN 201610243093
(43) Date of publication of application: 27.02.2019
(73) Proprietor: Wuhan Vision Pharmaceutical Technology Co., Ltd., Wuhan, Hubei 430000 (CN)
(72) Inventor: WU, Shiping, Wuhan Hubei 430000 (CN); XU, Hongyu, Wuhan Hubei 430000 (CN); HU, Xiangdong, Wuhan Hubei 430000 (CN)
(74) Representative: Zeuner Summerer Stütz
(86) International application number: PCT/CN2017/080337
(87) International publication number: WO 2017/181897

(56) References cited:
- CN-A- 104 994 844
- CN-A- 105 101 970
- CN-A- 105 801 610
- US-A1- 2014 194 385
- US-A1- 2014 194 386

## Description

### Technical field

The invention relates to the technical field of medicine, in particular to a novel broad spectrum beta-lactamase inhibitor.

### Background technology

Antibiotics, especially beta-lactam antibiotics (e.g., penicillin, cephalosporins, and carbapenems) have been one of the most effective tools for treating bacterial infectious diseases. All of these drugs have beta-lactams in their core molecular structure and generally exhibit broad spectrum efficacy against gram-positive and gram-negative bacteria by inhibiting bacterial cell wall synthesis. They are widely used in clinic because of their strong bactericidal action and low toxicity.

However, various pathogenic bacteria have evolved to produce beta-lactamases that can inactivate beta-lactams, and the ability to spread and share this tool between and within species. This enzyme inactivates the beta-lactam antibiotic by catalyzing the hydrolysis of the lactam ring, inactivating its binding to the target penicillin binding protein in the bacterium and producing drug resistance. This is the most important mechanism known to be associated with bacterial resistance. The broadly defined beta-lactamases are two basic classes of serine hydrolases and metallohydrolases. According to its amino acid sequence, β-lactamases are generally further classified into four classes: Ambler classes A, B, C and D [Bush and Jacobby, updated β-lactamase functional classification, antimicrobial agents and chemotherapy, 54 (3): 969-976 (March 2010)]. The active sites of class A, C and D enzymes are serine, while less class B enzymes are zinc-dependent. Traditionally, the class A is penicillin enzyme, the class C enzyme is cephalosporin enzyme, the class D enzyme is broad spectrum or extended spectrum β-lactamase (ESBL), and the class B enzyme also has broad spectrum resistance. One strategy to overcome this most important mechanism of bacterial resistance is to develop new antibiotics, such as newer generations of cephalosporins and carbapenems, that can escape the loss of early serine beta-lactamases. However, the reliance on beta-lactam antibiotics has led to a significant increase in recent diversification and incidence of novel beta-lactamases, such as class A extended spectrum beta-lactamases (ESBL) and carbapenem enzyme (KPC-2), class C chromosomes and plasmid-mediated cephalosporins (MPC, CMY), And class D oxacillinases (ESBL) and class B metal beta-lactamases (e.g. VIM, NDM). This significantly reduces the utility of the beta-lactam antibiotic family, including more recent beta-lactam drugs, such as carbapenems, which are the last means of clinical treatment, thus leading to serious medical problems and a major public health and economic burden [Llarrull et al., the future of beta-lactam classes, Current perspectives in microbiology, 13: 551-557 (2010).

Another strategy is to develop beta-lactamase inhibitors to help improve the effectiveness of beta-lactam antibiotics. The combination of inhibitors with β-lactam antibiotics can slow or prevent the degradation of β-lactam antibiotics and restore the sensitivity of β-lactamase producing bacteria to β-lactam antibiotics. However, new generation of cephalosporins and carbapenems are increasingly used to promote bacterial selection and transmission of mutations in new beta-lactamases, and that numb of serine-only beta-lactamases currently cataloged by the system has exceeded 750 variants (see Lahey Clinic website, Jacoby & Bush, "TEM, SHV and OXA broad spectrum and inhibitor-resistant beta-lactamase amino acid sequences." In many case, existing beta-lactamase inhibitor are not sufficient to combat that increasing diversity of beta-lactamases.

The three most common serine beta-lactamase inhibitors currently in commercial use, clavulanic acid, tazobactam and sulbactam, are only active against certain class a enzymes, and the active core of these inhibitors is still a beta-lactam loop, Their use leads to an increasing number of bacterial evolutions with a resistance mechanism against such beta-lactamase inhibitors, which severely limits their utility. In addition, a beta-lactamase inhibitor, such as Aonibactam, which has recently been approve by that FDA, Relebactam currently in clinical trials, and RPX7009, which is the same as the compound of the present invention, mainly act on enzymes of classes A and C. Minimal efficacy for class D and class B beta-lactamases [Bebrone et al., current challenges in antimicrobial chemotherapy: Focused beta-lactamase inhibition, pharmaceuticals, 70 (6): 651-679 (2010), or ineffective [Becker et al. Preclinical evaluation of the effect of carbapenem / beta-lactamase inhibitors (RPX2003 / RPX7009 combination on the serine-carbapenem enzyme producing pathogens tested, F-856, ICAAC Paper Summary, San Francisco (2012)). There is a considerable improvement in these new inhibitors over the current beta-lactamase inhibitors. As there are currently no D or B class β-lactamase inhibitors effective for human and animal infections, bacterial resistance to conventional antibiotics continues to rise. Therefore, it has become increasingly necessary to effectively deactivate all three classes of serine beta-lactamases and new metal beta-lactamases, as well as broad spectrum inhibitors that do not contain beta-lactam rings, as we have seen today.

The present available inhibitors are not sufficient to inhibit the increasing diversity of β-lactamases because of their narrow enzyme inhibitory activity or the presence of β-lactam rings in their structure. Today we urgently need novel broad spectrum β-lactamase inhibitors for the treatment of difficult to cure infectious diseases caused by drug-resistant bacteria. These bacteria produce extended spectrum and broad spectrum beta-lactamases (ESBL) of classes C, A, D and B, such as KPC-2 beta-lactamases of class A, which can even decompose carbapenems as a final means of clinical treatment of beta-lactam antibiotics. Therefore, there is a need to develop a novel broad spectrum beta-lactamase inhibitor which is used as a drug to inhibit the inactivation of beta-lactam antibiotics and restore antibacterial activity.

In recent years, it is a technical development direction that boric acid derivatives are used as a potential β-lactamase inhibitor for overcoming the problem of antibiotic resistance of bacterial β-lactam, which has achieved initial success. The present invention and other related patent documents reflect the exploration in this field (US2014/194385A1, Rempex Pharmaceuticals INC, 10 July 2014; US 2014/194386A1, VenatoRx Pharmaceuticals INC, 10 July 2014).

### Summary of the invention

It is an object of the present invention to solve the deficiencies of the above background art and to provide a novel broad spectrum beta-lactamase inhibitor which is used as a drug to inhibit the inactivation of beta-lactam antibiotics and restore antibacterial activity.

The technical scheme of the invention is as follows: Compounds of formula I: 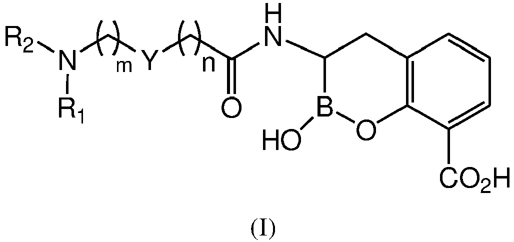
Where: m is 0, 1 or 2; n is 0 or 1; Y is disubstituted pyridyl-CsHsN-;
R₁, R₂ are hydrogen or amino, -C = NH (NH₂), hydroxyl, halogen carboxyl, cyano, thiol, optionally substituted C1-C5 alkyl, C1-C5 alkoxy, C1-C5 alkenyl, C3-C6 cycloalkyl, C3-C6 heterocyclic, thioether, sulfone.

According to the invention, the compound I has one of the following structures as recited in claim 1:

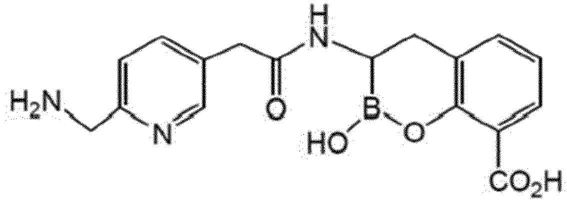

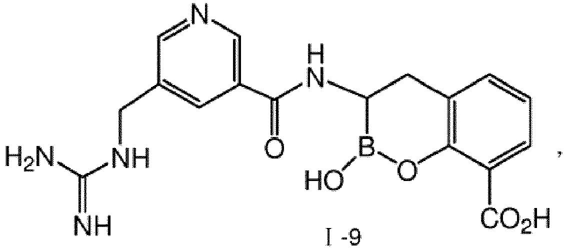

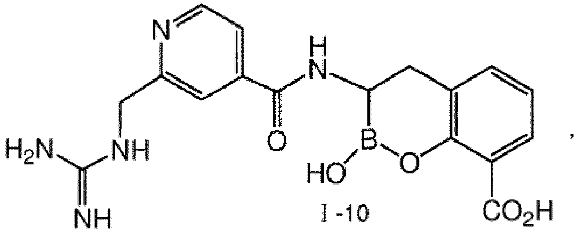

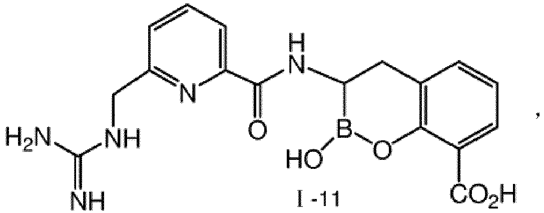

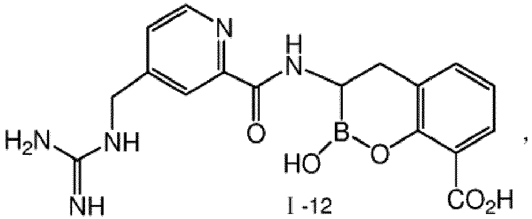

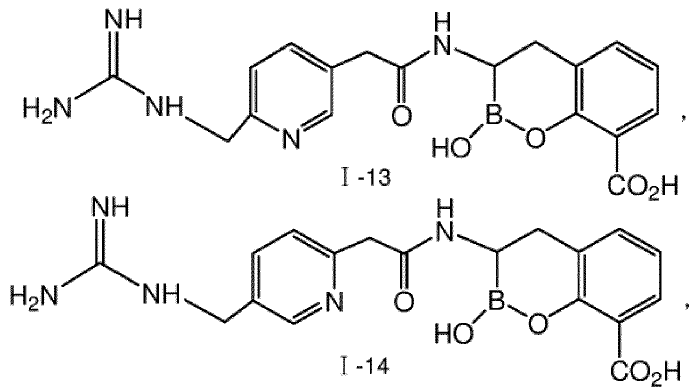

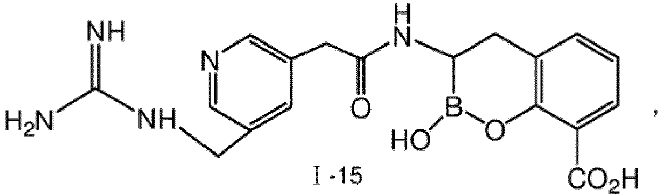

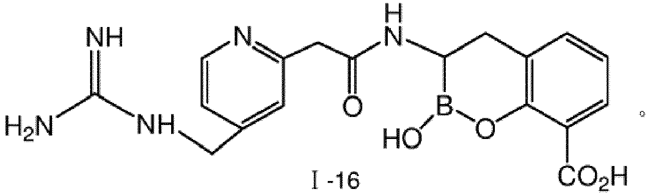

In addition, the invention also provides the use of the above compounds recited in claims 2 to 5. Specifically, the invention provides the use of any the above compounds as a beta-lactamase inhibitor in the preparation of a medicament for treating bacterial infection disease.

Preferably, the compound is made into a pharmaceutical type of injection, powder injection, oral agent, spray, capsule, suppository or any pharmaceutically acceptable excipient.

Further, the compound is used in conjunction with a beta-lactam antibiotic to prepare a medicament for the prevention or treatment of bacterial infections.

Further, a pharmaceutical composition of the compound, a beta-lactam antibiotic and optionally a pharmaceutically acceptable carrier is prepared as a medicament for the treatment of bacterial infections.

The compound I is synthesized by using a commercially available arylboronic acid using the general synthetic route described below.

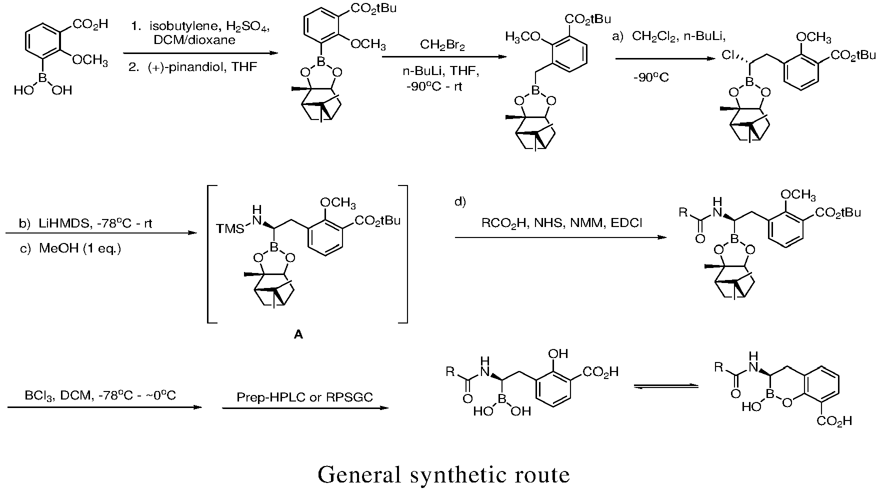

In the above synthesis scheme, firstly, in the presence of concentrated sulfuric acid, 2-methylpropene is used to protect the carboxylic acid group into tert-butyl ester, and then (+) -2,3-pinane diol is used to convert boric acid into chiral borate ester. The benzyl borate ester was then obtained by the homologous reaction of (bromomethyl) lithium (modified to Matteston et al., Organometallics, 1985, 4, 1687-1689). The conversion to the bis (trimethylsilyl) amine intermediate can be accomplished under the conditions described in the following documents: Schoichet et al., J. Am. Chem. Sue. 2003, 125, 685-695. These intermediates can then be converted to the desired amide by reaction with the active ester. The active ester can be obtained by reaction of a carboxylic acid with N-hydroxysuccinimide (NHS), 3-dimethylaminopropyl) -3-ethylcarbodiimide hydrochloride reagent (EDCI). The removal of pinane diol groups and the deprotection of carboxylic and phenolic groups can be accomplished in one step under acidic conditions, such as in a solution of dichloromethane containing a Lewis acid such as boron trichloride (BCl₃) or boron tribromide (BBr₃), or aluminum chloride (AlCl₃). Although those skilled in the art will appreciate that relatively small amounts of I- (S) isomers may be present in the reaction product, the applicant may obtain the dominant (more than 99%) I- (R) enantiomer according to the methods of the literature. Also, as shown in the synthesis scheme, these compounds having o-hydroxy groups on the aryl ring may be present as free carboxylic acids, or in the form of cyclic borates, or in a mixture of cyclic and open chain forms.

### The use and administration of β-lactamase inhibitors ;

The invention also provides the compound for use. When it is used in combination with β-lactam antibiotics, it strongly restores the antimicrobial activity of the β-lactam antibiotics against drug-resistant bacteria.

As described above, the compounds of formula (I) have a beta-lactamase inhibitory effect and are therefore used to inhibit beta-lactamases. As a particular embodiment of its use, the compounds of the present invention may be used in combination with antibiotics of other beta lactam antibiotics inactivated by the action of beta-lactamases to restore the activity of these antibiotics for use in the treatment of infections. Thus, according to one embodiment of the present invention, there is provided a beta-lactamase inhibitor and pharmaceutical composition comprising a compound of formula (I) as an active ingredient for use in combination with a beta lactam antibiotic. That is, the beta-lactamase inhibitors and pharmaceutical compositions of the present invention are administered to animals, including humans, simultaneously or sequentially with beta-lactam antibiotics.

Administration of a beta-lactamase inhibitor may be in any pharmacological form comprising a therapeutically active amount of a beta-lactamase inhibitor or further comprising a pharmaceutically acceptable carrier or additive such as a stabilizer, surfactant, plasticiser, solubilizer, brightener, thickener, lubricant, buffer, sweetener, Fragrance, fragrance, fragrance, sugar coating agent, flavoring agent, substrate, absorbent, binder, suspending agent, moisturizing agent, coating agent, moisture modifying agent, moisture modifying agent, filler, antifoaming agent, chewing agent, refrigerant, colorant, pH adjusting agent, softening agent, emulsifier, adhesive agent, adhesive agent, foaming agent, A dissolving agent, a liquefying agent, a dissolving accelerator, a dispersing agent of a dissolving accelerator, a dispersing agent, a blasting agent, a disintegrating agent, a disintegrating accelerator, an anti-wetting agent, a sterile agent, a preservative, an analgesic agent, or the like, or a combination of two or more of these additives. The person skilled in the art can select suitable additives for the formulation to prepare the desired form of the pharmaceutical composition according to widely used methods and forms of the pharmaceutical composition. Examples of additives include gelatin, lactose, refined sugar, titanium oxide, starch, corn starch, crystalline cellulose, hydroxypropyl methyl cellulose, carboxymethyl cellulose, hydroxypropyl cellulose, microcrystalline wax, white petrolatum, magnesium aluminum silicate, anhydrous calcium phosphate, magnesium stearate, citric acid, trisodium citrate, sorbitol, Fatty acid sorbitol ester, polysorbate (polysorbate), fatty acid sucrose ester, polyoxyethylene hydrogenated castor oil, vegetable oil, polyvinylpyrrolidone, light anhydrous silicic acid, pumice powder, benzyl alcohol, gum arabic, propylene glycol, polyglycol, cyclodextrin and hydroxypropyl cyclodextrin. The dosage regimen may be adjusted, for example the drug may preferably be administered once to several times a day at 0.1100 mg / kg to 1100 mg / kg (based on the weight of the compound and the route of administration) to provide the best therapeutic response. Therapeutic compositions or pharmaceutical compositions may be administered orally or non-enteric as is known in the art, and examples of orally administered formulations include tablets, granules, granules, powders, powders, syrups, solutions, capsules, chewable tablets or suspensions, and the like. Parenteral routes of administration include intranasal, intraocular, ear, transdermal, endotracheal, rectal, intraurethral, subcutaneous, intramuscular, intravenous, intrathecal or intracerebral administration, etc. Examples of preparations for parenteral administration include injections, drops, inhalers, sprays, suppository, vaginal suppository, transdermal absorbent, transmucosal absorbent, eye drops, ear drops, nasal drops or patches. Wherein a liquid formulation, such as an injection or a drop, may provide a pharmaceutical composition, for example in the form of a lyophilized powder, which may be dissolved or suspended in water or other suitable solvent, such as a physiological saline or glucose infusion, at the time of application. Beta-lactamase inhibitors may also be linked or conjugated with antibodies to transferrin receptors, polyethylene glycols, etc., to achieve the desired solubility, stability, half-life and other pharmaceutically advantageous properties. Beta lactam antibiotics include penicillins, cephalosporins, carbapenems, monocyclic lactams, bridged monocyclic lactams, or their prodrugs and combinations, which may be used in the form of pharmaceutically acceptable salts, such as sodium salts. Examples of penicillins include penicillin, penicillin V, ampicillin, amoxicillin, amoxicillin, carboxicillin, ampicillin, impicillin, epiracillin, tepicillin, cyclohexicillin, piroxicillin, meloxicillin, sulbacillin, piperacillin and other well-known penicillin. Illustrative example of cephalosporins include cefotaxime, ceftriaxone, ceftriaxone, ceftriaxone, ceftazidime, cefazolin, cefalexin, cefapilil, cefapilin, cefapilin, cefamandolena-phate, Cefradine, 4-hydroxycefalexin, cefoperazone, oxafenone, cefminox, fluoxofos, ceftazidime, ceftazidime, cefuroxime, cefotazolam, cefmetazole, cefpiroxil, cefazolin and other well-known cephalosporins are also described herein. Pecific examples of carbapenems include imipenem, meropenem, ertapenem, biapenem, doripenem, tebipenem, and the like.

The beta-lactamase inhibitor or a pharmaceutically acceptable salt thereof may be administered at the same time as the administration of the beta-lactam antibiotic, or separately. This can be carried out in the form of a mixture of two active ingredients or in the form of a pharmaceutical combination product of two independent active ingredients. A large number of strains producing beta-lactamases include, for example, Escherichia coli, Klebsiella pneumoniae, Pseudomonas aeruginosa, Bacillus cereus, Acinetobacter baumannii, Enterobacter cloacae, Bacteroides fragilis, Aeromonas hydrophila, Serratia mucosae, Stenosus, Xylose-oxidizing alkali-producing bacilli, Legionella grammoni pneumonia, meningeal pyogenic chrysobacteria, indole-producing chrysogenum bacilli and phloem citrate bacilli, and the like. The dosage of beta-lactamase inhibitors and their pharmaceutically acceptable salts and their ratio to beta-lactam antibiotics can also vary widely, for example in a weight ratio of 1: 0.5 to 1: 18. It should be understood that the amount of composition actually administered will be determined by the physician according to the relevant circumstances, including the disease to be treated; The selection of the composition to be administered; Age, body weight and response of individual patients; The severity of the patient's symptoms and the route of administration chosen.

Inhibition of bacterial growth the present disclosure also provides compounds to be used in a method of inhibiting bacterial growth by, for example, reducing bacterial resistance to beta lactam antibiotics, the method comprising contacting a bacterial cell culture with a beta-lactamase inhibitor as described herein. Bacteria that are inhibited by administration of the beta-lactamase inhibitors of the present invention are bacteria that are resistant or highly resistant to beta-lactam antibiotics. Experiments to inhibit beta-lactamase activity are well known in the art. For example, standard enzyme inhibition assays that determine the ability of a compound to inhibit beta-lactamase activity may be employed. The beta-lactamases used in such experiments may be prepared from recombinant DNA techniques, may also be purified from bacterial sources, or the sensitivity of known or engineered beta-lactamase producing bacteria to inhibitors may be determined. Other experiments on bacterial inhibition include agar plate diffusion and agar dilution. Beta-lactamases can thus be inhibited by contacting the beta-lactamase with an effective amount of the compound of the invention, or by contacting the bacteria producing the beta-lactamase with an effective amount of said compound, thereby bringing the beta-lactamase in the bacteria into contact with the inhibitor. Contact can occur in vitro or in vivo, enabling the inhibitor to bind to beta-lactamases. Inhibition includes reduction and elimination of beta-lactamase activity.

### The advantageous effects of the present invention are:

1. The compound of the present invention effectively solves the problems of inhibitors caused by diversity of β-lactamases produced by bacteria and failure of β-lactam antibiotics, and is a novel broad spectrum β-lactamase inhibitor.
2. Beta-lactamases have three major molecular types based on serine and one major molecular type based on metal and each type contains a significantly different number of beta-lactamase variants, and the compounds of the present invention show effective activity against all four major classes of beta-lactamases.
3. Through the study of the activity of the compound of the invention against four types of β-lactamases, it is found that the compound can successfully inhibit the activity of four types of β-lactamases (SHV-5, CTXM-15, KPC-2, VIM-2, P99 +, OXA-23), Moreover, when they are combined with β-lactam antibiotics, they have strong growth inhibitory activity against all kinds of bacteria producing β-lactamases, and can be expected to develop into a drug which can restore the antibacterial activity of β-lactam antibiotics to treat and prevent infections caused by bacteria in animals, including human beings, with great social and economic benefits.

### Specific implementation methods

The present invention will be further described in detail with reference to specific examples.

The general method for the synthesis of compound I is to prepare using arylboronic acid using the general synthetic route described below.

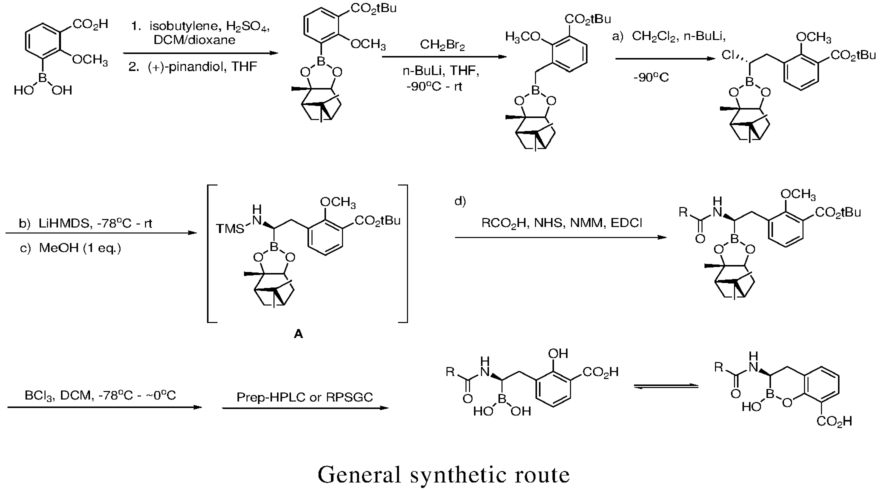

In the above synthesis scheme, firstly, in the presence of concentrated sulfuric acid, 2-methylpropene is used to protect the carboxylic acid group into tert-butyl ester, and then (+) -2,3-pinane diol is used to convert boric acid into chiral borate ester. The benzyl borate ester was then obtained by the homologous reaction of (bromomethyl) lithium (modified to Matteston et al., Organometallics, 1985, 4, 1687-1689). The conversion to the bis (trimethylsilyl) amine intermediate can be accomplished under the conditions described in the following documents: Schoichet et al., J. Am. Chem. Sue. 2003, 125, 685-695. These intermediates can then be converted to the desired amide by reaction with the active ester. The active ester can be obtained by reaction of a carboxylic acid with N-hydroxysuccinimide (NHS), 3-dimethylaminopropyl) -3-ethylcarbodiimide hydrochloride reagent (EDCI). The removal of pinane diol groups and the deprotection of carboxylic and phenolic groups can be accomplished in one step under acidic conditions, such as in a solution of dichloromethane containing a Lewis acid such as boron trichloride (BCl3) or boron tribromide (BBr3), or aluminum chloride (AlCl3). Although those skilled in the art will appreciate that relatively small amounts of I- (S) isomers may be present in the reaction product, the applicant may obtain the dominant (more than 99%) I- (R) enantiomer according to the methods of the literature. Also, as shown in the synthesis scheme, these compounds having o-hydroxy groups on the aryl ring may be present as free carboxylic acids, or in the form of cyclic borates, or in a mixture of cyclic and open chain forms.

### Example 1

### Synthesis of the Compound 1-1 ;

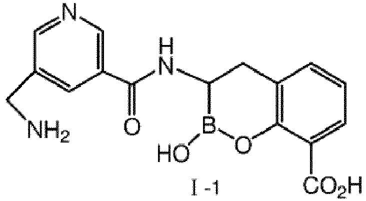

Compound I-1 is the specific structure of compound I when m = 1, n = 0, Y is 3,5-disubstituted pyridyl-CsHsN-, R₁ and R₂ are hydrogen.
I. Synthesis of (2-tert-butoxycarbonyl) -5-aminomethylpyridine-3-carboxylic acid, i.e. compound A, the synthetic route is as follows: 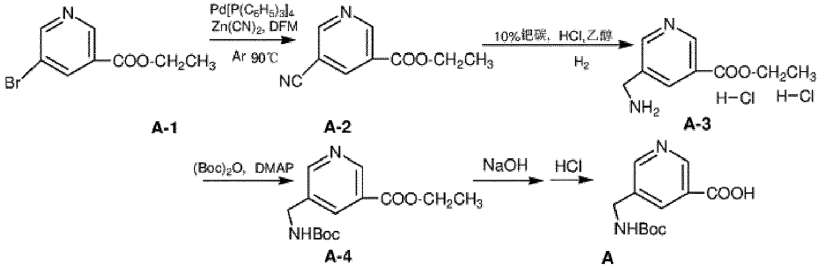
   Step 1. Synthesis of ethyl 5-cyanotinate (Compound A-2) charged with ethyl 5-bromonicotinate (Compound A-1,4.60 g, 20.0 mmol), zinc cyanide (9.94 g, 84.6 mmol), tetrad (triphenylphosphine) in a 300 ml round bottom flask. In 4.06 mmol) and DMF (100 ml). The mixture was heated at 90 °C. under argon for 15 hours. After cooling, the reaction was quenched with 10% ammonium acetate solution and extracted with ethyl acetate. The combined organic extracts were washed with water, brine and concentrated. The residue was purified by silica gel chromatography eluting with a gradient of 2 / 98 (V / V, volume to volume ratio) ethyl acetate / hexane to 10 / 90 (V / V) ethyl acetate / hexane to give product A-2: 3.36 g (92%). ESI-MS M / Z 177 (MH)^{+.}
   Step 2. Synthesis of 5-aminomethyl-ethyl nicotinate dihydrochloride (Compound A-3) ethyl 5-cyanotinate (Compound A-2, 3.88 g, 22.0 mmol), A solution of 10% palladium on carbon (2 g) and hydrochloric acid (15.7 ml, 4M in dioxane) was dissolved in ethanol (140 ml) and charged with 60 psi hydrogen (wherein 1 psi= 6894,76 Pa) and stirred in a Parr shaker for 4 hours. The mixture was filtered through Celite and the filtrate was concentrated to give 5.10 g of crude product (92%) Compound A-3. ESI-MS M / Z 181 (MH)^{+.}
   Step 3. Synthesis of 5-tert-butoxycarbonyl aminomethyl-ethyl nicotinate (Compound A-4) by adding (5-aminomethyl-ethyl nicotinate dihydrochloride (Compound A-3.5.08 g, 20.0 mmol) to a solution of 40 ml of unbutanol and 13 ml of acetone, Di-tert-butyl dicarbonate (13.05 g, 60.0 mmol), sodium bicarbonate (3.36 g, 40.0 mmol), and 4- (dimethylamino) pyridine (DMAP 5.10 mg, 4.15 mmol). The mixture was stirred overnight at room temperature. The reaction was quenched with saturated ammonium chloride and extracted three times with ethyl acetate. The combined organic extracts were washed with brine, dried and evaporated. The crude material was purified by silica gel chromatography, eluting from a gradient of 20 / 80 (V / V) ethyl acetate / hexane to 50 / 50 (V / V) ethyl acetate / hexane to give the product 5.60 g (100%) of Compound A-4 as a white solid: ESI-MS RN / Z 281 (MH)⁺.
   Step 4. Synthesis of (5-tert-butoxycarbonyl aminomethyl-nicotinic acid (Compound A)) to (5-tert-butoxycarbonyl aminomethyl-nicotinic acid ethyl ester (Compound A-4,5.6 g, To a mixed solution of methanol (20 ml) and water (10 ml) was added sodium hydroxide (10.5 g, 262 mmol) and the mixture stirred at room temperature for 15 hours during which time the solution became clear. The solvent was removed in vacuo and 3N hydrochloric acid was added dropwise with stirring and the pH adjusted to between 4 and 5. The solvent was removed in vacuo and the product purified by C18 reverse phase silica gel chromatography to give 5.56 g (100%) of compound A as a white solid. ESI-MS M / Z 253 (MH)⁺.
II. The main reaction, the synthesis of compound I-1, is as follows: 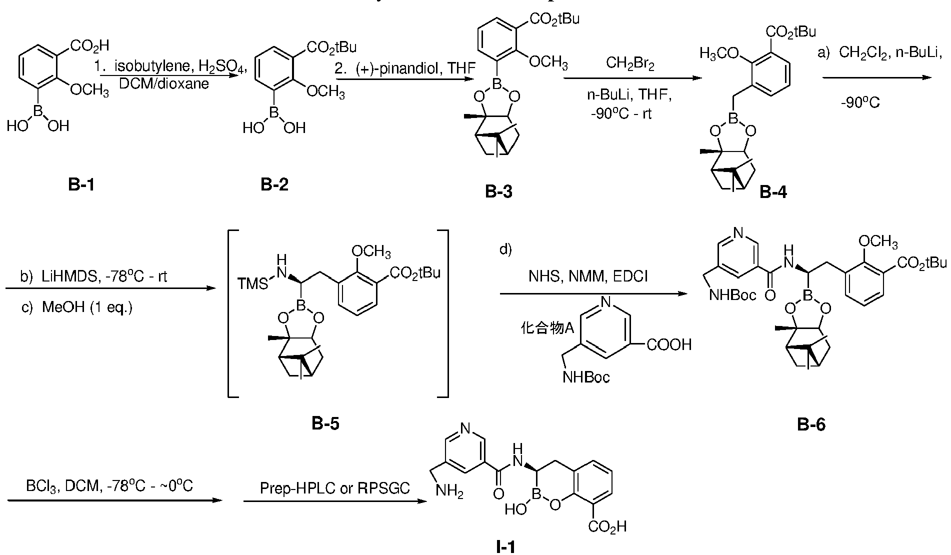
   Step 1. Synthesis of tert-butyl 3-dihydroxyboro-2-methoxybenzoate (Compound B-2) 3-dihydroxyboro-2-methoxybenzoic acid (Compound B-1,5.0 g, 25.5 mmol) added to 1,4-dioxane (30 ml) and sealed in a tube; Additional concentrated sulfuric acid (98%, 15 ml) was added. The solution was cooled to 0 °C. and an equal volume of isobutylene was bubbled in. The tube was sealed and stirred at room temperature for 18 hours. The solution was cooled in an ice bath, the seal was opened, and the solution was stirred at room temperature for 30 minutes. The solution was basified with saturated aqueous sodium bicarbonate solution and extracted twice with ethyl acetate. The combined organic layers were washed with water (5.times.), brine, dried over sodium sulfate and concentrated in vacuo to give the product 4.0 g (62%) of white solid compound B-2. ESI-MS M/Z 275 (M+Na)^{+.}
   Step 2. Synthesis of 2-methoxy-3- (2,9,9-trimethyl-3,5-dioxo-4-boro-tricyclic [6,1,1,02, 6] decyl-4-benzoic acid tert-butyl ester (compound B-3) -di-pinyl-70, 2. A solution of 15.9 mmol and tert-butyl 3-dihydroxyboryl-2-methoxybenzoate (compound B-2, 4.0 g, 15.9 mmol) in tetrahydrofuran (THF, 21 ml) was stirred at room temperature for 15 h. The solution was concentrated in vacuo and the residue was washed with hexane to obtain 5.0 g (86%) of a slowly crystallized white solid, ESI-MS M/Z 409 (M+Na)⁺.
   Step 3. Synthesis of 2-methoxy-3- (2,9,9-trimethyl-3,5-dioxo-4-boro-tricyclic [6,1,1,0^{2,6}] decyl-benzoic acid (compound B-4)) -tert-butyl ester in liquid nitrogen at -90 °C, Tert-butyl 2-methoxy-3- (2,9,9-trimethyl-3,5-dioxo-4-boro-tricyclic [61.1.0^{2,6}] decyl-4-yl) benzoate (compound B-3,8.5 g, 22 mmol) and dibromomethane. To a solution of 26.4 mmol of THF (65 ml), n-butyllithium (10.56 ml, 2.5 M in hexane, 26.4 mmol) was added over 10 minutes. The solution was stirred at -90 °C for 45 minutes. The reaction was gradually warmed and stirred overnight. The reaction was quenched with water and extracted with ethyl acetate. The combined organic layers were washed with water and brine, dried over magnesium sulfate and concentrated in vacuo. The residue was purified by silica chromatography eluting with a 70% dichloromethane / hexane-30% hexane gradient to obtain 8 g of product compound B-4 (91% yield) as a colorless oil. ESI-MS M/Z 401 (MH)⁺.
   Step 4. Synthesis of 3- (2R) - [2- [(5-tert-butoxycarbonyl methyl-nicotine) -amino] 2- (2,9,9-trimethyl-3,5-dioxo-4-borazine-tricyclic [6,1, 1, 0^{2,6}], N-BuLi (10.5 ml) was added to a solution of tetrahydrofuran (65 mL) added with anhydrous methylene chloride (1.80 ml, 28.50 mmol) at -90 °C. and within 10 minutes to t-butyl 4-4-yl-ethyl-2-methoxybenzoate (compound B-6). Hexane solution of 2.5M, 26.3 mmol. The solution was stirred at -90 °C and 2-methoxy-3-(2,9,9-trimethyl-4,5-dioxo-4-boro-tricyclic [6,1,1,0,^{2,6}] decyl-4-methyl-77-benzoic acid (8 g) was added by syringe within 20 minutes. Solution of 21.93 mmol in THF (11 ml). After 30 minutes the cold trap was removed and the mixture warmed to 0 °C and kept stirring for 1 hour. The solution was then cooled to -78 °C and lithium bis (trimethylsilyl) amino (LHMDS, 1.0 M THF solution, 24.1 ml) was added over 5 minutes. The reaction was gradually warmed and stirred overnight. The mixture was then cooled to -10 °C and anhydrous methanol (0.96 ml 24.1 mmol) was added. It was stirred for 45 minutes and then the ice bath was removed and the solution stirred for 1.25 h at room temperature. At this stage LCMS shows 2-methoxy-3- (2-(2,9,9-trimethyl-3-5-dioxo-4-boro-tricyclic [6,1,0^{2,6}]) decyl to -4-butyl-2-(trimethylsilyl-5-amino) -ester. All solvent was then removed in vacuo and the residue dissolved in 140 ml of DCM for use.
      The prepared (5-tert-butoxycarbonyl aminomethylnicotinic acid (compound A, 5.53 g, 21.93 mmol)) was dissolved in dichloromethane (220 ml) in a separate dry round bottom flask. The solution flask was cooled to 0 °C. and NMM (7.3 ml, 66.4 mmol), N-hydroxysuccinimide (NHS) (5.09, 44.2 mmol): 1- (3-dimethylaminopropyl) -3-ethylcarbodiimide hydrochloride (EDCI) was added. And 44.2 millimoles (mmol). The mixture was stirred at 0 °C for 30 minutes and then at room temperature for one hour. To this reaction mixture was added a stand-by solution (DCM solution of compound B-5) at 0 °C. The cooling bath was removed and the reaction was quenched with water after stirring at room temperature for 1.5 hours, the aqueous phases were extracted with dichloromethane, the combined organic layers were dried over sodium sulfate and concentrated in vacuo. The crude product was purified by flash column chromatography of silica gel eluting with a gradient of 30 / 70 (v / v) ethyl acetate / hexane to 70 / 30 (v / v) ethyl acetate / hexane to yield 4.36 g (30%) of compound B-6, slightly yellow. ESI-MS M / Z 664 (MH)^{+.}
   Step 5. Synthesis of (R) 3- (5-aminomethyl) nicotinamide-2-hydroxy-3,4-dihydro-2H-benzo [1,2] dioxaborane-8-carboxylic acid hydrochloride (compound 1-1) [2 [2-tert-butyrylpyridyl-9-carbox-Methyl-3,5-dioxa-4-boron-tricyclic [6,1,1,0^{2,6}] decyl-4-yl) ethyl-2-methoxybenzoic acid tert-butyl (compound B-6,4.30 g, 6.48 mmol) is dissolved in dichloromethane (15 ml). Under argon, a solution of boron trichloride (65 ml, 65 mmol, 1M solution in dichloromethane) at -78 °C was added. After stirring the mixture at -78 °C for 1 hour, LC-MS indicated consumption of the starting material at which time the reaction was quenched with water (30 ml) at 0 °C. The methylene chloride layer was evaporated by rotation. Additional 30 ml of water was added and the aqueous layer was extracted with ether (3.times.30 ml). After concentration of the aqueous phase, purified by chromatography on C18 reverse phase silica gel to give 1.40 g (61%) of a white solid of compound I-1. ESI-MS M / Z 342 (MH-H₂O)⁺.

### Example 2

### Synthesis of the Compound I-2 ;

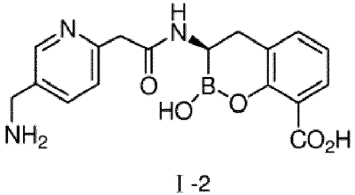

Compound I-2 is the specific structure of compound I when m = 1, n = 1, Y is 2,5-disubstituted pyridinyl-CsHsN-, R₁ and R₂ are all hydrogen.
I. Synthesis of 6-tert-butoxycarbonyl aminomethyl-pyridine-3-yl-acetic acid, i.e. compound C, the synthetic route is as follows: 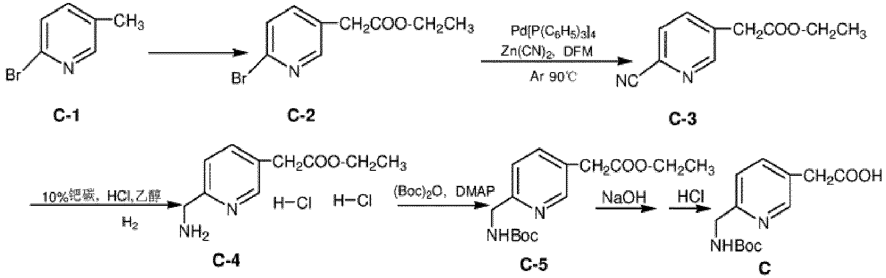
   Step 1. Synthesis of (6-bromo-pyridine-3-yl) -ethyl acetate (compound C-2) in a 500 mL round bottom flask, diisopropylamine (13.2 ml 93.92 mmol) mixed with tetrahydrofuran (41 ml) and cooled to -78 °C . And n-butyllithium (2.5 M in hexane; 38 ml, 91.20 mmol) was added and the mixture was stirred for 30 minutes. Then 2-bromo-5-methylpyridine dissolved in 17 ml of tetrahydrofuran (compound C-1,5 ml, 46.92 mmol) was added. And the mixture was stirred for 2 hours. Diethyl carbonate (6.2 ml, 51.40 mmol) was then added and the mixture stirred overnight while gradually warming to room temperature. The reaction was quenched with saturated ammonium chloride and extracted three times with ethyl acetate. The combined organic extracts were washed with brine, dried and concentrated. Purification by silica gel chromatography eluting with a gradient of 2 / 98 (V / V) to 7 / 93 (V / V) ethyl acetate / hexane gave the product 8.05 g (70%) of compound C-2 as a colorless oil. ESI-MS M / Z 246 (MH)⁺.
   Step 2. Synthesis of (6-cyano-pyridine-3-yl) -ethyl acetate (compound C-3) charged with 6-bromo-pyridine-3-ethyl acetate (compound C-2, 4.91 g 20.0 mmol), zinc cyanide (9.94 mol), Tetra (triphenylphosphine) palladium (0) (4.69 g, 4.06 mmol) and DMF (100 ml). The mixture was heated under argon at 90 °C for 15 hours. After cooling, the reaction was quenched with 10% ammonium acetate solution and extracted with ethyl acetate. The combined organic extracts were washed with water, brine and concentrated by drying, purified by chromatography on silica gel, eluting with a gradient of 2 / 98 (v / v) ethyl acetate / hexane to 10 / 90 (v / v) ethyl acetate / hexane to afford 3.45 g of compound C-3 (90.3%). ESI-MS M / Z 191 (MH)⁺.
   Step 3. Synthesis of (6-aminomethyl-pyridine-3-yl) ethyl acetate dihydrochloride (compound C-4) 6-cyano-pyridine-3-yl ethyl acetate (compound C-3,4 g, 21.03 mmol) 10% palladium carbon (2 g), And hydrochloric acid (15.7 ml of 4M in dioxane) were added to ethanol (140 ml) and loaded into a Parr shaker and charged with 60 psi hydrogen (wherein 1 psi = 6894,76 Pa). Stirring for 4 hours, the mixture was filtered through Celite and the filtrate was concentrated to give 5.13 g (91%) of the crude compound C-4. ESI-MS M / Z 195 (MH)⁺.
   Step 4. Synthesis of (6-tert-butoxycarbonyl-pyridine-3-yl) -ethyl acetate (compound C-5) by adding (6-aminomethyl-pyridine-3-yl) -ethyl acetate dihydrochloride (compound C-4, 5.13 g 19.2 mmol) in 40 ml of unbutanol and 13 ml of acetone, Di-tert-butyl dicarbonate (12.98 g, 59.5 mmol) was added with sodium bicarbonate (3.23 g 38.4 mmol) and 4- (dimethylamino) pyridine (5.13 mg, 4.20 mmol). The mixture was stirred overnight at room temperature, the reaction was quenched with saturated ammonium chloride and extracted three times with ethyl acetate, the combined organic extracts were washed with brine, dried and concentrated, the crude material was purified by silica gel chromatography, eluting with 20 / 80 (v / v) to 30 / 70 (v / v) ethyl acetate / hexane gradient gave the product 6.02 g (100%) of compound C-5 as a white solid: ESI-MS M / Z 295 (MH)⁺.
   Step 5. Synthesis of (6-tert-Butoxycarbonyl Aminomethyl-Pyridine-3-yl) -Acetic Acid (Compound C)
      To (6-tert-butoxycarbonyl-aminomethyl-pyridine-3-yl) ethyl acetate (compound C-5,6 g, 20.3 mmol) in a mixed solution of methanol (20 mL) and water (10 mL) was added sodium hydroxide (1.05 g) and 2.62 mmol. The mixture was stirred for 15 hours during which time the solution became clear. The methanol was removed in vacuo and 3N hydrochloric acid was added dropwise with stirring to give a pH between 4 and 5. After concentrated solution. Purification by means of carbon 18 phase silica gel chromatography gave 5.42 g (100%) of compound C as a white solid: ESI-MS M / Z 267 (MH)⁺.
II. The main reaction, the synthesis of compound I-2, is as follows: 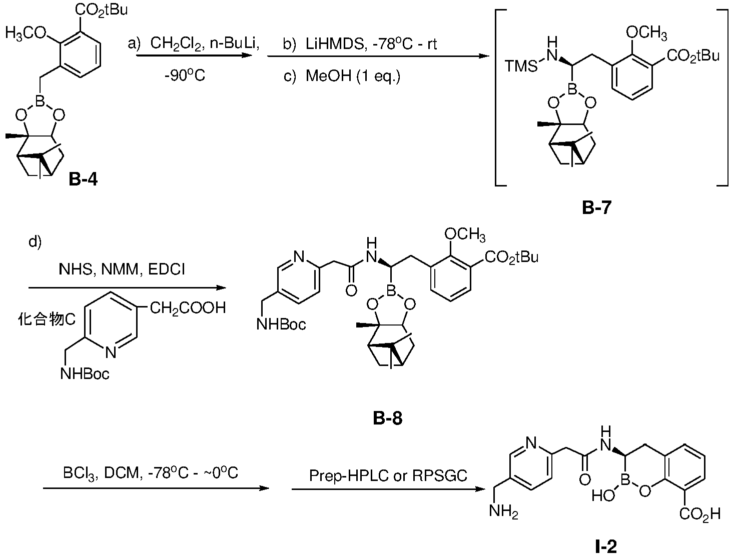
   Step 1. Synthesis of 3- [2- [(5-tert-butoxycarbonyl-pyridine-3-ylacetyl) -amino] 2- (2,9,9-trimethyl-3,5-dioxo-4-tricyclic [6,1,02,⁶] -tert-butyl-n-butyl) N-butyllithium (10.5 ml, 2.5 M hexane solution, 26.3 mmol) was added to a solution of tetrahydrofuran (65 mL) added with anhydrous dichloromethane (1.80 ml, 28.50 mmol) at -90 °C for 15 minutes. The solution was stirred at -90 °C. and 2-methoxy-3-(2,9,9-trimethyl-3,5-dioxo-4-boro-tricyclic [6,1,02,⁶] decyl-4-methyl-77-isotert-benzoate was added by syringe within 20 minutes. A solution of 21.93 mmol in THF (11 ml) and compound B-4 were prepared in the same manner as in Example 1. After 30 minutes the cold trap was removed and the mixture warmed to 0 °C. and kept stirring for 1 hour. The solution was then cooled to -78 °C. and lithium bis (trimethylsilyl) amino (LHMDS, 1.0 M THF solution, 24.1 ml) was added over 5 minutes. The reaction was gradually warmed and stirred overnight. The mixture was then cooled to -10 °C. and anhydrous methanol (0.96 ml 24.1 mmol) was added. It was stirred for 45 minutes and then the ice bath was removed and the solution stirred for 1.25 h at room temperature. At this stage LC-MS shows 2-methoxy-3- (2-(2,9,9-trimethyl-3-5-dioxo-4-boro-tricyclic [6,1,02,⁶] decyl-4-yl) -2-(trimethylsilylamino) -2. All solvent was then removed in vacuo and the residue dissolved in 140 ml of DCM for use.
      Prepared (6-tert-butoxycarbonyl aminomethyl-pyridine-3-yl-acetic acid (compound C, 55.84 g, 22.0 mmol)) was dissolved in DCM (220 ml) in a separate dry round bottom flask. The solution flask was cooled to 0 °C and NMM (7.3 ml, 66.4 mmol), N-hydroxysuccinimide (NHS) (5.09 g, 44.2 mmol): 1- (3-dimethylaminopropyl) -3-ethylcarbodiimide hydrochloride (EDCI) was added. And 44.2 millimoles (mmol). The mixture (DCM solution of compound B-7) was stirred at 0 °C for 30 minutes and then at room temperature for one hour. A stand-by solution was added to the reaction mixture at 0 °C. The cooling bath was removed and the reaction was quenched with water after stirring at room temperature for 1.5 hours, the aqueous phases were extracted with dichloromethane, the combined organic layers were dried over sodium sulfate and concentrated in vacuo. The crude product was purified by flash column chromatography of silica gel eluting with a 30 / 70 (v / v) ethyl acetate / hexane gradient to 70 / 30 (v / v) ethyl acetate / hexane to give 2.84 g (20%) of product compound B-8, yellowish. ESI-MS M / Z 678 (MH)^{+.}
   Step 2. Synthesis of (2R) 3- {2- {2- [(6-aminomethyl-pyridine-3-yl-acetyl) -amino] -2-hydroxyboronethyl} -2-hydroxy-benzoic acid hydrochloride (compound I-2) 3-2 [(6-tert-butylamino-picolyl-) Tert-butyl 2,9,9-trimethyl-3,5-dioxa-4-boro-tricyclic [6.1.1.0^{2,6}] decyl-4-yl] -2-methoxy-benzoic acid (compound B-8,2.48g, 3.66 mmol) was dissolved in dichloromethane. Boron trichloride (38 ml, 38 mmol, 1M solution in DCM) was added under argon at -78 °C. The mixture was stirred for 1 hour. The reaction was quenched with water (30 ml) at 0 °C. The rotary evaporation to methylene chloride. And added to water (20ml). And the aqueous layer (3.times.30 ml) was extracted with ether. After aqueous phase concentration, the product was purified by C18 reverse phase silica gel chromatography to give 770 mg (58%) of a white solid compound I-2. ESI-MS M / Z 356 (MH-H₂O)^{+.}

### Performance testing of the compounds prepared as described above as β-lactamase inhibitors ;

Experimental Methods for Analysis of β-Lactamases. Isolation of beta-lactamases: Crude beta-lactamase extract was prepared from 20 ml of culture shaken overnight. Escherichia coli cells containing SHV5 or CTXM15, Enterobacter cloacae containing P99, Klebsiella pneumoniae containing KPC2, Escherichia coli cells containing either SHV5 or CTXM15, Acinetobacter baumannii containing oxa-23 and pseudomonas aeruginosa containing vim-2 were diluted 10-fold, and grown at 37 °C. in Mueller Hinton II (MHII) broth to logarithmic mid-term (OD at 600 nm, .5-.8). The cells were precipitated at 5000 g, washed and resuspended in 2 mL of PBS at pH 7.0. Beta-lactamases were extracted by four cycles of freezing, thawing and subsequent centrifugation. The activity of β-lactamase in the extract was determined by using the chromogenic cephalosporin cefotizol. The amount of protein in each of the beta-lactamase preparations was determined by a biscinchoninic acid (BCA) experiment. Beta-lactamase inhibition: To determine the level of beta-lactamase inhibition, the compound was diluted in PBS at pH 7.0 to obtain a concentration of 1000 µM to 005 µM in a microplate. An equal volume of diluted enzyme stock was added and the plates were incubated at 37 °C for 10 minutes. Then, the solution of cefonitrogen as a substrate was dispensed into each well at a final concentration of 100 µM, and the plate Plus384 (high flux microplate spectrophotometer) was used; Molecular Devices Corp., Syvale, Calif., used a dynamic program to immediately read at 486 nm for 10 minutes. The maximum rate of metabolism was then compared to the control well (without inhibitor) and the percentage of enzyme inhibition was calculated for each concentration of inhibitor. The inhibitor concentration (IC50) required to reduce the initial hydrolysis rate of the substrate by 50% was calculated using the Software SoftMax Pro 5.0 (Molecular Devices Corp.) at 486 nm as the residual activity of beta-lactamases. The ability of the embodiments of the present invention to inhibit beta-lactamase was evaluated using the methods described above. The results of these experiments are summarized in representative enzymes spanning different subtypes (where shv5 and ctxm15 are different subtypes of ambler class a extended spectrum beta-lactamase, kpc2 is class a carbapenem enzyme, vim-2 represents class b metalloenzyme, p99 represents chromosome class c, Whereas OXA-23 belongs to class D representative enzymes) in Table 1, where A represents an IC₅₀ > 1 µM, B represents an IC₅₀ of 0.1 to 1 µM, and C represents an IC₅₀ of <0.1µM.

**Table 1. Half inhibitory concentration IC₅₀ (µM) range for various β-lactamase activities of the example compounds:**

| An example of implementation | Class A | | | B class | C class | D class |
|---|---|---|---|---|---|---|
| | SHV-5 | CTXM-15 | KPC-2 | VIM-2 | P99+ | OXA-23 |
| One | C | C | C | C | C | C |
| Two | C | C | C | C | C | C |
| Clavulanic acid control group | C | C | A | A | A | A |

In vitro antibacterial experiment of inhibition of β-lactamase. In order to determine that ability of the test compound to inhibit the growth of beta-lactamase-producing bacterial strain, traditional cell-based screening experiments were employed. Six bacterial strains producing beta-lactamases were used: Escherichia coli expressing class a extended spectrum beta-lactamases (esbl) ctxm15 and shv5 (e.coli), Klebsiella pneumoniae expressing class a carbapenem enzyme kpc2, Pseudomonas aeruginosa expressing class B VIM-2, Enterobacter cloacae expressing class C P99 +, and Acinetobacter baumannii expressing class D OXA-23 were studied. To evaluate the ability of the test compound to inhibit beta-lactamase activity, an improved broth microdilution experiment was used. This experiment was performed in Cation Adjusted Mueller Hinton Broth (CAMHB, BD # 212322, BD Diagnostic Systems, Sparks, MD). The bacterial strains were grown in CAMBH broth for 35 hours. All six strains were grown in the presence of 50 µg / mL ampicillin to ensure that resistance was maintained. At the same time, the test compound was diluted into 0.1 mg / mL stock solution in DMSO. The compound was added to the microplate and diluted in a 2-fold series in CAMHB to a final concentration of 32 µg / mL to 0.25 µg / mL. A cover layer containing cephalosporin CAMHB was added to the compound at a termination concentration of 8 µg / mL or 16 µg / mL Ceftazidime (CAZ, Sigma # C38091G, Sigma Aldrich, St. Louis, MO) of 8 µg / mL was used as a partner antibiotic for the following strains: Escherichia coli expressing class A ESBL SHV5 (MIC alone > 1024 µg / mL), Klebsiella pneumoniae (MIC = 32 µg / mL) expressing carbapenem class A KPC2 and Enterobacter cloacae (MIC = 256 µg / mL) expressing class C P99 + AmpC; Ceftazidime 16 µg / mL (CAZ) was used as a partner of the following strains: Acinetobacter baumannii expressing class D enzyme OXA-23 (MIC > 256 µg / mL), Pseudomonas aeruginosa expressing class B enzyme VIM-2 (MIC > 256 µg / mL); And 8 µg / mL cefotaxime (TAX, USP # 1097909, U.S. Pharmacopeia, Rockville, MD) was used as a co-antibiotic for E. coli expressing class A ESBLCTXM15 (MIC = 1024 µg / mL). The MIC reading of the dose escalation test of the test compound indicates the concentration of the analyte sufficient to inhibit beta-lactamase activity and protect the original antibacterial activity of cephalosporins. Six plates are required for each compound and each plate is used for one bacterial strain. In addition to the dose escalation test of the test compound, the MIC of a group of cephalosporins was tested to ensure that the strain remained consistent in behavior between the test and the test. Once the test compound and cephalosporin are added, the plates may be inoculated. The inoculation was carried out according to the clsi broth microdilution method. After inoculation, the plates were incubated at 37 °C. for 16-20 hours and then the minimum inhibitory concentration (MIC) of the test compound was visually determined. The ability of the embodiments of the present invention to inhibit the growth of beta-lactamase producing bacteria in the presence of beta-lactam antibiotics was evaluated using the methods described above. Representative results are shown in Table 2, where A represents MIC > 16 µg / mL, B represents MIC = 2-16 µg / mL, and C represent MIC < 1 µg / mL .

**Table 2 broad spectrum inhibition of bacterial growth, MIC ranges of compounds in the presence of fixed amounts of cephalosporin antibiotics,**

| An example of implementation | Class A and Class A, | | | B class | C class | D class |
|---|---|---|---|---|---|---|
| | Escherichia coli SHV-5+ CAZ (8µg / mL) | Escherichia coli CTXM-15+ TAX (8µg / mL) | klebsiella pneumoniae KPC-2+ CAZ (8µg / mL) | Pseudomonas aeruginosa VIM-2+ CAZ (16 µg / mL) | Enterobacter cloacae P99+ + CAZ (8µg / mL) | Acinetobacter Bauman OXA-23+ CAZ (16 µg / mL) |
| One | B | C | C | B | C | B |
| Two | C | C | C | B | C | B |
| Clavulanic acid control | C | C | B | A | A | A |

## Claims

1. A compound, selected from the group consisting of the following structures:

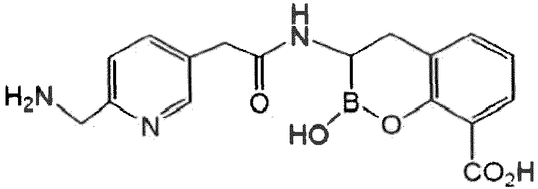

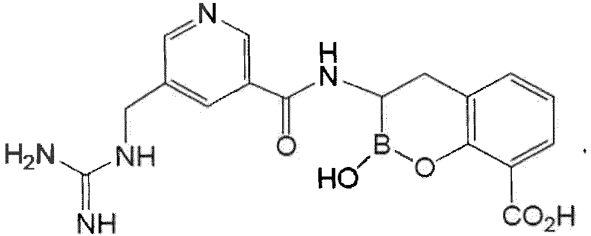

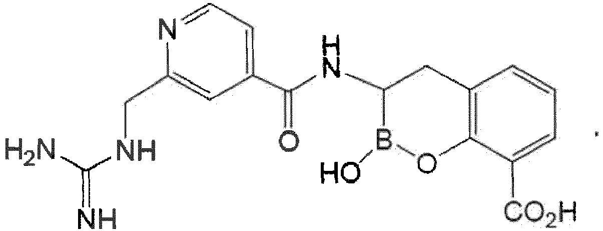

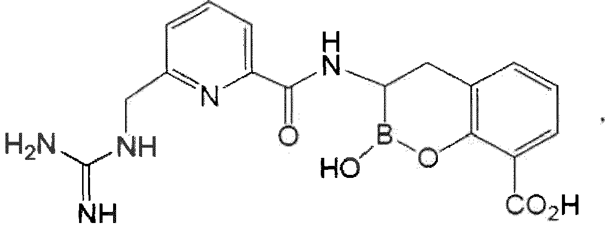

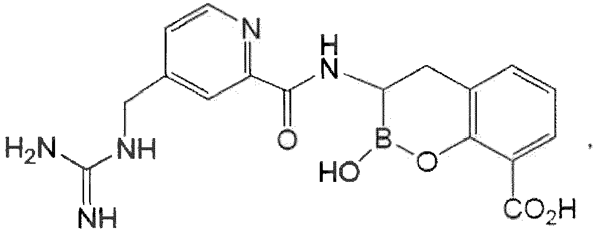

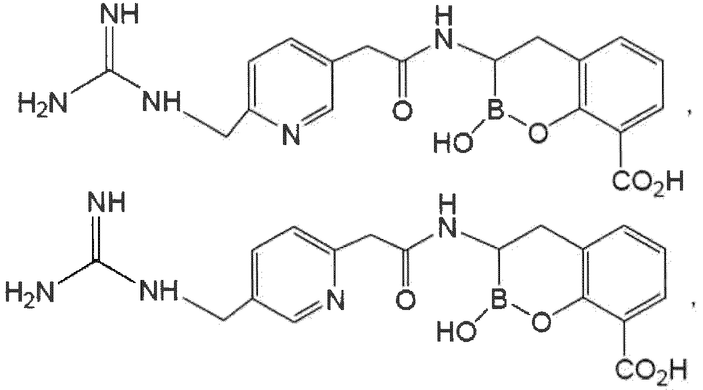

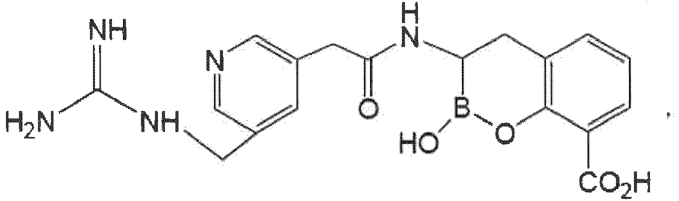

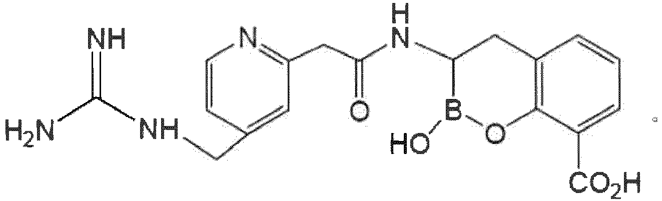

2. The use of the compound according to claim 1 as a beta-lactamase inhibitor in the preparation of a medicament for the treatment of bacterial infections.

3. The use of the compound according to claim 2, **characterized in that** the compound is made into a drug type of injection, powder injection, oral agent, spray, capsule, suppository or any pharmaceutically acceptable excipient.

4. The use of the compound according to claim 2, **characterized in that** the compound is used together with a beta-lactam antibiotic to prepare a medicament for the prevention or treatment of bacterial infections.

5. The use of the compound according to claim 4, said compound, a pharmaceutical composition of a beta-lactam antibiotic and optionally a pharmaceutically acceptable carrier to prepare a medicament for the treatment of bacterial infections.

## Patentansprüche

1. Verbindung, die ausgewählt ist aus der Gruppe, bestehend aus den folgenden Strukturen:

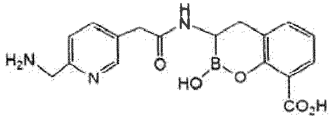

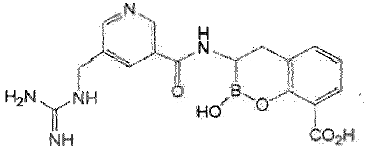

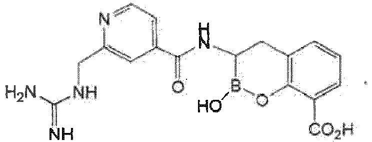

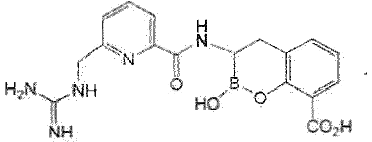

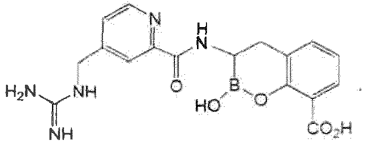

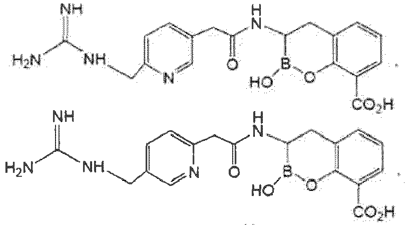

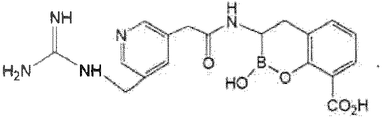

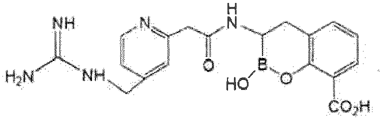

2. Verwendung der Verbindung nach Anspruch 1 als Beta-Laktamase-Inhibitor bei der Herstellung eines Arzneimittels zur Behandlung bakterieller Infektionen.

3. Verwendung der Verbindung nach Anspruch 2, **dadurch gekennzeichnet, dass** die Verbindung zu einem Arzneimittel vom Typ Injektion, Pulverinjektion, orales Mittel, Spray, Kapsel, Zäpfchen oder einem beliebigen pharmazeutisch verträglichen Hilfsstoff verarbeitet wird.

4. Verwendung der Verbindung nach Anspruch 2, **dadurch gekennzeichnet, dass** die Verbindung zusammen mit einem Beta-Laktam-Antibiotikum zur Herstellung eines Arzneimittels zur Vorbeugung oder Behandlung bakterieller Infektionen verwendet wird.

5. Verwendung der Verbindung nach Anspruch 4, dieser Verbindung, einer pharmazeutischen Zusammensetzung aus einem Beta-Laktam-Antibiotikum und wahlweise eines pharmazeutisch verträglichen Trägers zur Herstellung eines Arzneimittels zur Behandlung bakterieller Infektionen.

## Revendications

1. Composé, sélectionné dans le groupe constitué des structures suivantes :

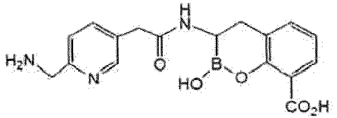

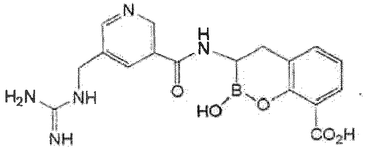

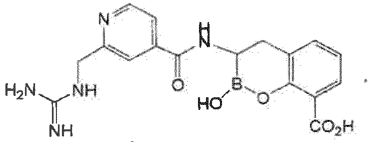

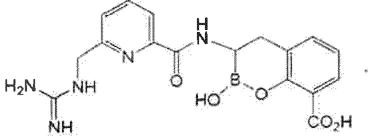

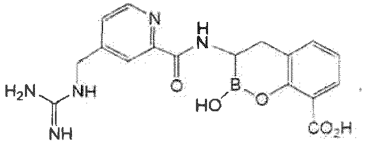

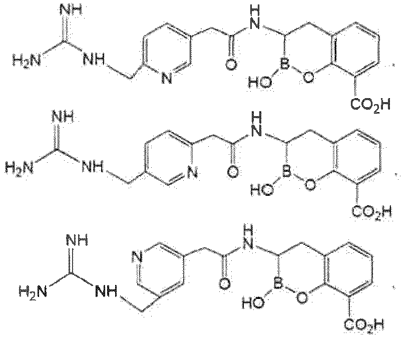

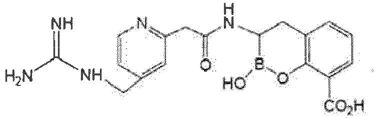

2. Utilisation du composé selon la revendication 1 en tant qu'inhibiteur de la bêta-lactamase dans la préparation d'un médicament pour le traitement d'infections bactériennes.

3. Utilisation du composé selon la revendication 2, **caractérisée en ce que** le composé est préparé en un type de médicament d'injection, d'injection de poudre, d'agent oral, de pulvérisation, de gélule, de suppositoire ou tout autre excipient pharmaceutiquement acceptable.

4. Utilisation du composé selon la revendication 2, **caractérisée en ce que** le composé est utilisé conjointement à un antibiotique bêta-lactame pour préparer un médicament pour la prévention ou le traitement d'infections bactériennes.

5. Utilisation du composé selon la revendication 4, dudit composé, d'une composition pharmaceutique d'un antibiotique bêta-lactame et facultativement d'un vecteur pharmaceutiquement acceptable pour préparer un médicament pour le traitement d'infections bactériennes.
